# FASCICULE DE BREVET EUROPEEN

(11) **EP 3 092 297 B1**
(45) Date de publication et mention de la délivrance du brevet: **19.06.2019**
(21) Numéro de dépôt: 15702538.8
(22) Date de dépôt: 08.01.2015
(51) Int. Cl.: C12M 1/00, C12Q 1/04, D06M 23/08

(54) **PROCÉDÉ DE DÉTECTION, IDENTIFICATION ET ÉNUMÉRATION DE MICRO-ORGANISMES DANS UN SUPPORT POREUX IMPRÉGNÉ À SEC PAR UN MILIEU RÉACTIONNEL DÉSHYDRATÉ**
VERFAHREN ZUR DETEKTION, IDENTIFIZIERUNG UND ZÄHLUNG VON MIKROORGANISMEN IN EINEM PORÖSEN, MIT EINEM ENTWÄSSERTEN REAKTIONSMEDIUM IMPRÄGNIERTEN TRÄGER
METHOD FOR DETECTING, IDENTIFYING AND ENUMERATING MICRO-ORGANISMS IN A POROUS SUPPORT DRY-IMPREGNATED WITH A DEHYDRATED REACTION MEDIUM

(30) Priorité: 09.01.2014 FR 1450149
(43) Date de publication de la demande: 16.11.2016
(73) Titulaire: Biomérieux, 69280 Marcy l'Étoile (FR)
(72) Inventeur: MONTET, Marie-Pierre, F-69200 Venissieux (FR); ROZAND, Christine, F-69290 St Genis les Ollières (FR)
(86) Numéro de dépôt international: PCT/FR2015/050035
(87) Numéro de publication internationale: WO 2015/104501

(56) Documents cités:
- WO-A1-03/093819
- WO-A1-2005/038123
- WO-A1-2005/061013
- WO-A1-2009/082667
- WO-A1-2010/001043
- WO-A2-02/02245
- FR-A1- 2 268 076
- US-A1- 2001 012 537
- US-A1- 2013 089 887
- GINA E. FRIDLEY ET AL: "Controlled release of dry reagents in porous media for tunable temporal and spatial distribution upon rehydration", LAB ON A CHIP, vol. 12, no. 21, 2012, page 4321, XP055147408, ISSN: 1473-0197, DOI: 10.1039/c2lc40785j
- JAMES H. JORGENSEN ET AL: "Antimicrobial Susceptibility Testing: A Review of General Principles and Contemporary Practices", CLINICAL INFECTIOUS DISEASES, vol. 49, no. 11, décembre 2009 (2009-12), pages 1749-1755, XP055034346, ISSN: 1058-4838, DOI: 10.1086/647952
- JEANNETE ZURITA ET AL: "Diagnosis and susceptibility testing of methicillin-resistant Staphylococcus aureus in Latin America", THE BRAZILIAN JOURNAL OF INFECTIOUS DISEASES, vol. 14, décembre 2010 (2010-12), pages 97-106, XP055187332, ISSN: 1413-8670, DOI: 10.1590/S1413-86702010000800005

## Description

La présente invention concerne, de façon générale, le domaine de l'analyse microbiologique. Plus particulièrement, elle porte sur un procédé de détection, identification et/ou énumération de micro-organismes dans un support poreux imprégné à sec dans toute son épaisseur par un milieu réactionnel déshydraté.

Dans les domaines du diagnostic clinique et du contrôle microbiologique industriel agroalimentaire, pharmaceutique ou cosmétique, les milieux de culture gélifiés en boite de Petri, le plus souvent gélosés, constituent depuis la fin du XIXème siècle un outil indispensable à la détection et à l'identification des micro-organismes pathogènes.

Plusieurs produits ont été proposés commercialement pour remplacer un milieu de culture en boite de Petri. L'un d'eux, le système Petrifilm™, comprenant des nutriments réhydratables, est très largement utilisé. Un autre système développé par la société Nissui Pharmaceutical, le Compact Dry™, consiste également en un milieu déshydraté. Ces milieux de culture ont pour avantage de se conserver plus longtemps qu'un milieu de culture gélosé prêt à l'emploi. Ils peuvent également, comme le pétrifïlm™, présenter un faible encombrement et ainsi utiliser un faible espace d'incubation.

Ainsi, il y a schématiquement deux façons pour obtenir un milieu de culture réhydratable :
La première consiste à apporter le milieu de culture sous une forme liquide dans le support puis à sécher l'ensemble.
La seconde consiste à coller le milieu de culture sous forme déshydraté à un support, afin d'obtenir d'emblée un milieu de culture réhydratable.

La première méthode, à savoir l'obtention de milieux nutritifs réhydratables fabriqués en incluant un phase d'imprégnation humide des nutriments, a fait l'objet de plusieurs demandes de brevets. Ainsi, la demande de brevet CN102337324, décrit un procédé dans lequel le bouillon nutritif est mélangé à un composant chimique dont l'évaporation est rapide. Le document WO2005/061013 décrit quant à lui un marqueur mis en solution dans un solvant et déposé sur une couche absorbante afin de détecter les vaginites. Plus récemment, la demande de brevet US20130089887. décrit un support, à savoir une fine membrane imprégnée de substrats chromogéniques et/ou fluorogéniques dissous dans un solvant, mis en contact avec un milieu gélosé. Le document FR 2268076 divulgue un materiau stable d'essai de sensibilité aux antibiotiques.Néanmoins, cette méthode, par la mise en solution dans l'eau ou dans un solvant, impacte négativement la durée de conservation du milieu de culture réhydratable. En effet, la mise en suspension de certains produits fragiles, tels que les enzymes, les substrats enzymatiques ou métaboliques, les antibiotiques, peut impacter sévèrement leur stabilité globale. Le chauffage nécessaire au séchage du milieu de culture peut également dénaturer et rendre inefficace les composants thermosensibles du milieu réactionnel. Cette méthode en phase aqueuse ne permet pas non plus de pouvoir maitriser et faire varier la localisation du milieu réactionnel et/ou des différents additifs nécessaires à la révélation bactérienne.

Afin de pallier les inconvénients des milieux de cultures obtenus par cette méthode, la deuxième méthode propose de déposer directement sur le support la poudre nutritive sans phase antérieure de mise en solution de cette dernière.

Ainsi, la société 3M propose un milieu nutritif déshydraté collé et déposé sur un film sans passer par une phase préalable de solubilisation du milieu. Ce dispositif est constitué de deux parties, un film inférieur et un film supérieur recouverts en surface par certains composants du milieu de culture deshydraté. Au moment de l'analyse, l'échantillon est déposé entre ces deux films.

Ce dispositif et la méthode de détection associée tels que décrits dans la demande WO 2009/082667, présentent plusieurs inconvénients.

Tout d'abord, ce dispositif nécessite pour sa fabrication une étape d'adhésion des nutriments deshydratés sur les films qui ont dû être préalablement encollés. Ensuite, le milieu de culture ne peut pas former de facto une structure tridimensionnelle dont on peut faire varier la hauteur, et la concentration par strates puisqu'il est collé à un film. Seule, une faible couche superficielle de milieu est donc disponible. Le volume de l'échantillon liquide nécessaire à l'analyse microbiologique ne peut alors excéder 1 ou 2 ml ce qui impacte le seuil de sensibilité de détection. Ensuite, le packaging du petrifilm™ nécessite la fabrication conjointe du film inférieur et du film supérieur. Il ne permet pas non plus, d'avoir sur un même dispositif plusieurs milieux de cultures différents. De plus, ce dispositif reste limité dans ses applications et ne peut, par exemple, être utilisé en tant qu'écouvillonnages ou pansements. Et enfin, le Petrifilm™ nécessite obligatoirement l'aide d'un opérateur extérieur apportant l'échantillon aqueux.

La publication de Gina Fridley et al. « controlled release of dry reagents in porous media for tunable temporal and spatial distribution upon rehydratation" Lab Chip (2012, November 7), vol. 12(21), pages 4321-4327, enseigne des méthodes d'impression de réactifs sur un support poreux.

D'autre part, dans une demande de brevet antérieur FR1257047, la demanderesse propose une méthode d'isolement à partir d'un échantillon à analyser, sur un milieu de culture réhydratable in situ, qui permet d'obtenir, des colonies isolées. Ce milieu rehydratable est recouvert d'une membrane permettant la réalisation de l'isolement des colonies. Ainsi le milieu de culture reste stérile et les colonies se développent sur la membrane qui se trouve juste au dessus de lui.

Or l'isolement de colonies sur un support gélosé ou non, est parfois vu comme une contrainte et est souvent incompatible avec des expérimentations effectuées en dehors du laboratoire et/ou avec des personnes ayant peu de connaissance et de savoir-faire en microbiologie.

Eu égard à la somme des problématiques développées supra, la présente invention propose un nouveau procédé de détection, identification et énumération de micro-organismes susceptibles d'être compris dans un échantillon.

Ainsi, un objectif de la présente invention est de fournir un dispositif comprenant un milieu deshydraté améliorant la sensibilité de détection.

Un autre objectif de la présente invention est de fournir un procédé de détection, identification et énumération de micro-organismes sans recourir à l'isolement.

Un autre objectif de la présente invention est de fournir un dispositif et un procédé permettant une multi détection, et ainsi obtenir à partir d'un même échantillon à analyser et sans réaliser d'isolement, des cultures isolées, identifiables, dénombrables sur différents milieux réactionnels présents sur un même dispositif.

L'invention a également comme objectif de fournir un dispositif et un procédé particulièrement flexibles. Le milieu réactionnel peut être un milieu plus ou moins complexe, chromogénique par exemple, ou très simple, c'est à dire contenant uniquement un nombre limité de substrats (antibiotiques, substrats métaboliques...). Le dispositif et le procédé peuvent également avoir des modalités d'utilisation très variées telles qu'un écouvillon ou un absorbant révélateur de contaminations microbiennes dans des pansements, des couches culottes, des conditionnements d'aliments.

Un autre objectif de la présente invention est de fournir un dispositif dont l'utilisation est possible par des personnes ayant peu de savoir-faire en microbiologie. Ainsi le dispositif peut être rehydraté en une seule fois par l'opérateur au moment de l'analyse de l'échantillon. La réhydratation peut également être réalisée in situ sans l'aide de l'opérateur si notamment l'échantillon à tester est placé à proximité du milieu réactionnel réhydratable en permettant sa réhydratation progressive. L'échantillon à analyser peut par exemple être une plaie exsudative ou un morceau de viande et produire un liquide susceptible de contenir les micro-organismes à détecter.

Un autre objectif de la présente invention est de fournir un dispositif qui sert lui même à la collecte de l'échantillon tels qu'un écouvillon.

Un objectif de la présente invention est de fournir un dispositif dont la production et la commercialisation sont facilitées par le fait que le support poreux imprégné à sec par un milieu réactionnel est produit indépendamment de son packaging.

Un autre objectif de la présente invention est de fournir un dispositif dans lequel un gradient de concentration des substrats réactionnels est effectué, permettant ainsi de limiter la quantité de ces substrats et de limiter le coût de production du dispositif.

D'autres objectifs apparaîtront à la lecture de la présente demande.

La présente invention vise donc à atteindre tout ou partie des objectifs précités.

En conséquence, la présente invention a pour objet un procédé de détection et/ou identification et/ou énumération, d'au moins un microorganisme cible dans un échantillon susceptible de le contenir, comprenant les étapes suivantes :
(a) fournir un dispositif pour la détection et/ou l'identification et/ou l'énumération de microorganismes comprenant un support poreux comprenant de la poudre d'un milieu de culture avec au moins un antibiotique et/ou au moins un composé thermosensible tel qu'un substrat enzymatique ou métabolique, dans toute son épaisseur, ledit support poreux ayant été imprégné à sec dans toute son épaisseur par ledit milieu de culture,
(b) mettre en contact un échantillon avec le support poreux,
(c) incuber le dispositif,
(d) détecter et/ou identifier et/ou énumérer la ou les colonies de microorganismes au sein du support poreux, lorsque les microorganismes recherchés sont présents dans l'échantillon.

Selon l'invention, le dispositif est imprégné à sec dans toute son épaisseur par un milieu réactionnel déshydraté. L'incorporation de matériaux pulvérulents dans des supports poreux peut être effectuée selon au moins quatre techniques :
- utilisation d'une pompe à vide telle que décrite dans le brevet US 5,213,843;
- La mise en vibration mécanique du support poreux lui-même sur lequel on a déposé la poudre par un système vibrant quelconque, les secousses permettant de faire pénétrer plus ou moins profondément la poudre;
- l'utilisation d'un champs électrostatique;
- La mise en vibration ultrasonore, simultanément avec l'application de la poudre, en utilisant un générateur Ultra Sons qui fait vibrer une sonotrode telle que décrit dans la demande de brevet FR 2866578. Lorsque le produit poreux défile sous la sonotrode, l'action de celle-ci met les particules de poudre en vibration, et celles-ci pénètrent alors dans les cavités du corps poreux.

De façon préférentielle, le procédé de fabrication du support poreux imprégné à sec dans toute son épaisseur comprend une étape de mise en vibration des particules de poudre par un champ électrique. Préférentiellement, ce champ électrique est alternatif. Le brevet EP 1.028.836 décrit l'imprégnation de textiles (non tissés, tissus...) en appliquant un champ électrique alternatif entre deux systèmes d'électrodes entre lesquels se trouve le textile recouvert de poudre. Les particules de poudre qui se chargent électriquement entrent en vibration à la fréquence du champ alternatif. Ainsi, de façon surprenante, cette technique peut être utilisée pour imprégner à sec un support poreux avec un milieu réactionnel déshydraté. Les déplacements des particules permettent donc leur pénétration dans les porosités du support. Les particules ont pénétré en profondeur le support poreux, dans toute l'épaisseur du support.
Ainsi, les zones du support imprégnées de milieu sont imprégnées dans toute l'épaisseur du support, la poudre ayant traversé l'épaisseur du support poreux. Ainsi, au moins tout ou partie du support poreux comprend un milieu réactionnel sous forme de poudre dans toute son épaisseur, certaines zones sur le support pouvant être néanmoins exempte de tout milieu comme par exemple le pourtour du support.

Le degré d'imprégnation des particules dans l'épaisseur du support peut être maitrisé, par exemple homogène, localisé ou en gradient, en fonction des caractéristiques des matières en présence (supports et poudres) mais aussi des caractéristiques du procédé mis enjeu (intensité du champ électrique, temps de traitement, fréquence....). Le support peut être imprégné de façon séquentielle dans le temps ce qui peut permettre une meilleure imprégnation. Ainsi, l'imprégnation de gélifiant peut avoir lieu préalablement à l'imprégnation de milieu réactionnel.

L'imprégnation à sec d'un milieu deshydraté dans toute l'épaisseur d'un support poreux ne nécessite pas d'utilisation d'eau et permet une maitrise de la localisation des particules. De plus, elle permet une flexibilité dans l'épaisseur de la couche imprégnée en permettant de définir différentes zones dans l'épaisseur de quantité et natures différentes.
De façon avantageuse un gradient de concentration des substrats réactionnels est effectué dans le support poreux, permettant ainsi de limiter la quantité de ces substrats et de limiter le coût de production du dispositif. Il peut être choisi également d'avoir un support poreux comprenant un type de substrat réparti de façon homogène et un autre type de substrat réparti en gradient. De façon avantageuse, le support est imprégné dans toute son épaisseur par un milieu nutritif et superficiellement par des substrats chromogènes. Dans un autre mode de réalisation, les substrats sont encapsulés permettant leur relargage séquentiel après l'incubation du dispositif. Ainsi ce mode de réalisation a l'avantage de limiter l'utilisation des substrats en évitant leur dilution dans le milieu nutritionnel lors de la réhydratation du milieu.
De même un agent sélectif comme un antibiotique peut également être encapsulé. Ce mode de réalisation est particulièrement avantageux car il permet de différer la mise en contact du contenu (comprenant une faible quantité de micro-organismes cibles, si ces derniers sont présents) avec l'agent sélectif destiné à orienter la croissance des micro-organismes vers celle des micro-organismes recherchés. Ainsi les micro-organismes en phase de stress microbien ne sont pas directement mis en contact avec l'agent sélectif, ce dernier risquant, à ce stade, soit de ralentir leur croissance et donc d'augmenter le temps nécessaire à l'analyse, soit d'inhiber totalement leur croissance et, ainsi, empêcher leur détection/identification. En effet, les micro-organismes cibles sont dits « stressés » lorsqu'ils sont présents dans l'échantillon à analyser. Les micro-organismes (parmi lesquels les micro-organismes cibles) ont besoin d'un certain laps de temps pour s'adapter aux conditions existantes à l'intérieur du support poreux. Dans leur état dit « stressé », les micro-organismes cibles sont particulièrement sensibles, notamment à la présence d'agents sélectifs tels que des antibiotiques.

Ainsi, le support poreux peut comprendre différents milieux réactionnels. Ces milieux réactionnels sont localisés en différentes zones du support. Ces zones peuvent correspondre à des zones verticales et donc à l'épaisseur du support et/ou correspondre à des zones horizontales du support. Le support poreux peut donc avoir au niveau d'une zone plusieurs milieux réactionnels tel que par exemple un milieu de culture et un milieu de révélation. Le dispositif peut également avoir un ou plusieurs milieux réactionnels disposés dans des zones différentes de un ou plusieurs supports poreux, chacune de ces zones ayant du milieu réactionnel distribué dans toute l'épaisseur du support.

En pratique, plusieurs paramètres peuvent influencer la mise en oeuvre de ce procédé comme principalement :
- la texture du réseau de fibres ou de filaments, ou d'une manière générale du support poreux utilisé ;
- les caractéristiques physico-chimiques des poudres telles que la nature ou la granulométrie de la poudre ;
- la durée de traitement, l'intensité du champs électrique ainsi que la fréquence du champ électrique ;

Il conviendra donc d'adapter ces paramètres afin de permettre une imprégnation à sec satisfaisante du milieu réactionnel dans le support poreux.

Préférentiellement, la quantité de milieu réactionnel, sous forme de poudre, imprégné dans le support poreux est comprise entre 0,01g/cm³ et 0,1g/cm³ de préférence entre 0,02g/cm³ et 0,09g/cm³, plus préférentiellement entre 0,03g/cm³ et 0,06g/cm³. Préférentiellement, lorsque le milieu réactionnel comprend un milieu de culture et éventuellement un milieu de révélation, la quantité de milieu réactionnel, sous forme de poudre, imprégné est comprise entre 0,01g/cm³ et 0,09g/cm³, plus préférentiellement entre 0,03g/cm³ g et 0,06 g/cm³. Ainsi la présente invention a pour avantage de permettre une croissance optimisée notamment due à la quantité en excès du milieu culture qui pallie ainsi les problèmes de compétition nutritionnelle entre les microorganismes.

Il est également décrit que lorsque le milieu réactionnel comprend un milieu de révélation sans milieu de culture, la quantité de milieu réactionnel, sous forme de poudre, imprégné est beaucoup plus faible et est comprise entre comprise 0,10mg/cm³ et 10mg/cm³.

Selon l'invention, le support poreux est mis en contact avec l'échantillon.

Dans un mode de réalisation de l'invention, l'échantillon est aqueux et va permettre la réhydratation du milieu réactionnel compris dans le support poreux.

Selon un autre mode de réalisation, un volume approprié de liquide est ajouté à l'échantillon et/ou au support poreux afin de réhydrater le milieu réactionnel, lorsque l'échantillon n'est pas aqueux ou insuffisamment aqueux.

En pratique, l'homme du métier choisira le volume approprié de liquide ou de l'échantillon aqueux en fonction de sa viscosité, du diamètre du support poreux, ceci afin de réhydrater le milieu et permettre la croissance des micro-organismes. Avantageusement, la réhydratation du support poreux nécessite un volume de liquide ou d'échantillon aqueux supérieur à 2ml, préférentiellement supérieur à 3ml, encore préférentiellement supérieur à 4ml, ce qui permet d'améliorer la sensibilité de détection lorsque les microorganismes sont en faible concentration dans l'échantillon.

Selon la présente invention, l'échantillon peut comprendre une étape préalable de préparation, concentration ou dilution de l'échantillon.

Selon l'invention, la réhydratation du support peut être réalisée avec ou sans l'intervention d'un opérateur.

L'échantillon aqueux peut être ajouté manuellement à l'aide d'une pipette ou automatiquement dans le dispositif. Il peut également être contenu dans au moins un réservoir intégré au dispositif et/ou des canaux permettant la réhydratation du support poreux. Il se répand alors dans le support par simple pression du réservoir. Avantageusement, l'échantillon est mis en contact avec le support poreux en le plaçant en dessous du support poreux. Ainsi, la réhydratation a lieu par la partie inférieure et/ou latérale, de préférence par la partie inférieure. Ce mode opératoire permet une hydratation homogène de tout le support poreux et évite, notamment, aux nutriments et/ou substrats d'être entrainés par le liquide ou l'échantillon aqueux, dans la partie inférieure du dispositif. Avantageusement, ce mode opératoire permet au procédé selon l'invention d'être effectué dans l'espace en solutionnant le problème lié à l'absence de gravité de l'échantillon et/ou du liquide.

Dans un mode de réalisation, il n'y a pas d'intervention humaine et l'échantillon aqueux provient directement d'une zone productrice du liquide à tester. Il peut s'agir par exemple d'une plaie exsudative, de denrées alimentaires relarguant des liquides pendant leur stockage. L'échantillon, de par sa nature, va libérer une partie de ses liquides constitutifs qui vont au cours du temps imbiber le support poreux. La zone productrice de l'échantillon à tester peut également être une zone périnéale des humains ou animaux excrétant de l'urine. Le support poreux est alors mis à proximité de cette zone et est imprégné progressivement par l'échantillon aqueux produit.

Dans un autre mode de réalisation, la mise en contact de l'échantillon avec le support poreux se fait par prélèvement de l'échantillon à l'aide du support poreux. Le support poreux est alors utilisé comme écouvillon et l'opérateur devra placer ce dernier dans un tube contenant la quantité adéquat de liquide si l'échantillon n'est pas aqueux ou insuffisamment aqueux.

Le dispositif est ensuite incubé in situ (cas du pansements, de la couche culotte..) ou en incubateur durant un laps de temps suffisant pour permettre la détection des colonies microbiennes au sein de du support poreux.

Selon un mode de réalisation préféré, le procédé selon l'invention est un procédé de détection qui peut être mis en oeuvre par lecture visuelle ou optique du support poreux.

L'invention porte également sur un dispositif comprenant un support poreux imprégné à sec dans toute son épaisseur par un milieu réactionnel déshydraté permettant la révélation de colonies de microorganismes à l'intérieur dudit support, ledit support poreux étant calandré.

Le support poreux a été imprégné à sec dans toute son épaisseur, c'est-à-dire que lorsqu'il y a présence d'un milieu réactionnel à un endroit du support, il est présent au niveau de cette zone dans toute l'épaisseur du support.

Le support poreux a subi une opération de calandrage. Le calandrage, par la pression et la température de chauffe générées, permet la rétention et le maintien stable dans le temps du milieu réactionnel déshydraté dans le support poreux, en assurant la rétention des différents éléments tels que les éléments nutritifs dans le support poreux. Il permet également l'obtention d'une surface supérieure rigoureusement lisse et plane du support poreux.

Préférentiellement le calandrage se fait à une température supérieure à la température ambiante, préférentiellement à une température comprise entre 30°C et 60°C. Une température inférieure à 60°C permet de ne pas dénaturer les composés thermolabiles. Le calandrage permet, outre l'accélération de la réhydratation du support poreux par rapport à support poreux non-calandré, une compression des fibres constituant le support poreux. Cette compression, associée à la présence du milieu déshydraté au sein du support poreux permet une rehydratation simultanée dans toutes les zones du support placées horizontalement et préserve ainsi la répartition choisie (homogène ou en gradient) des substances.

Le calandrage permet ainsi de maintenir le milieu réactionnel à l'intérieur du support et sa manipulation aisée.

Le support comprend au moins un milieu réactionnel déshydraté, sous forme de poudre, réparti dans toute l'épaisseur du support poreux, ledit support poreux ayant une épaisseur comprise entre 0,5 à 2 mm après calandrage. Préférentiellement, le support poreux a une épaisseur de 0,8 mm à 1,8 mm, encore préférentiellement de 1 à 1,5 mm. La surface du support poreux est comprise entre 1 cm² et 40 cm² préférentiellement entre 10 cm² et 30 cm², plus préférentiellement entre 15 cm² et 25 cm².

Le support poreux est apte à retenir un volume d'eau supérieur à 2 ml, préférentiellement supérieur à 3 ml. Ainsi, un support poreux de 25 cm² de surface et une épaisseur de 1 mm après calandrage aura la capacité à retenir un volume d'eau de 3 ml.

Le dispositif selon l'invention, par la présence de milieu de culture dans toute son épaisseur présente donc l'avantage de permettre une meilleure sensibilité par sa capacité à retenir un volume d'échantillon liquide élevé.

Préférentiellement, la quantité de milieu réactionnel, sous forme de poudre, imprégné dans le support poreux est comprise entre 0,01g/cm³ et 0,1g/cm³ de préférence entre 0,01g/cm³ et 0,09g/cm³, plus préférentiellement entre 0,03g/cm³ et 0,06g/cm³. Préférentiellement, lorsque le milieu réactionnel comprend un milieu de culture et éventuellement un milieu de révélation, la quantité de milieu réactionnel sous forme de poudre imprégné est comprise entre 0,01g/cm³ et 0,09g/cm³, plus préférentiellement entre 0,03g/cm³ g et 0,06 g/cm³. Ainsi le présente invention a pour avantage de permettre une croissance optimisée notamment due à la quantité en excès du milieu culture qui pallie ainsi les problèmes de compétition nutritionnelle entre les microorganismes. De façon surprenante, la détection de certaines souches est ainsi plus rapide que sur milieu gélosé.

Le dispositif selon l'invention comprend au moins un antibiotique et/ou au moins un composé thermosensible tel qu'un substrat enzymatique ou métabolique. Il présente ainsi l'avantage d'avoir une stabilité accrue du fait que ce composé n'est pas été mis en suspension au cours de la fabrication du dispositif et/ou subit de chauffage nécessaire à la déshydratation d'un milieu de culture.

Selon un mode de réalisation, le dispositif comprend un support poreux comprenant au moins un milieu réactionnel, sous forme de poudre, réparti de façon homogène dans l'épaisseur du support poreux. Selon un autre mode de réalisation, au moins un milieu réactionnel, sous forme de poudre, est réparti de façon graduelle dans l'épaisseur du support poreux.

Un autre mode de réalisation propose au moins deux milieux réactionnels différents, sous forme de poudre, répartis en au moins deux couches, ledit support comprenant à un point donné de l'épaisseur, l'un ou/et l'autre milieu de culture.

Selon l'invention, le dispositif comprend une pluralité de milieux réactionnels.

De façon préférentielle, le dispositif comprend une couche supérieure protectrice. La couche protectrice est disposée sur le support poreux et aucune autre couche n'est disposée entre le support poreux et la couche protectrice. De préférence la couche protectrice est placée directement sur le support poreux.

La couche protectrice peut être translucide ou transparente permettant la visibilité des colonies au travers de cette couche. Elle permet également d'éviter les contaminations au cours de l'incubation. Elle est imperméable aux bactéries et limite la perte de vapeur d'eau. En effet, le dispositif est incubé pendant un temps et à une température prédéterminés permettant la croissance des micro-organismes indépendamment des conditions d'humidité ambiante. Ainsi, la nature de la couche supérieure est choisie de façon à permettre les échanges gazeux nécessaires à la croissance des micro-organismes tout en permettant une hydratation locale.

Préférentiellement, le dispositif comprend un réceptacle pour contenir l'échantillon aqueux et/ou le liquide. De préférence, le réceptacle comprend également le support poreux qui est alors préférentiellement réhydraté par sa partie inférieure.

Avantageusement, le dispositif comprend une couche inférieure imperméable à l'eau. De préférence cette couche inferieure est rigide, permettant une meilleure prise au main du dispositif. Elle est fabriquée à partir de composés tels que le polyester, polypropylène, polystyrène. De préférence, elle est fabriquée à partir de cellulose. Il peut s'agir de carton ou de papier associé à un film imperméable à l'eau. Elle peut contenir des canaux thermoformés qui serviront à la bonne réhydratation du support poreux.

De façon avantageuse, les différentes couches du dispositif sont fabriqués à partir de matériaux recyclables.

Selon un mode de réalisation particulier de l'invention, la couche inférieure est translucide ou transparente.

Selon un mode de réalisation particulier de l'invention, le dispositif comprend également un code d'identification tels que les code-barres ou les étiquettes RFID.

Selon la présente invention, il est aussi possible d'associer au sein d'un même dispositif différents milieux réactionnels disposés les uns à côté des autres et qui seront réhydratés simultanément avec le même échantillon à tester.

Dans un mode de réalisation, le dispositif comprend une tige à l'extrémité de laquelle le support poreux, imprégné à sec par un milieu réactionnel déshydraté, est fixé.

La fixation peut être réalisée par tout moyen connu de l'homme du métier comme par exemple le collage ou encore thermosoudage.

Le support poreux fixé à une tige peut ainsi jouer le rôle d'écouvillon. Avantageusement, le dispositif comprend également un tube transparent imperméable à l'eau pouvant recueillir l'écouvillon et un bouchon hermétique du tube.

Après réalisation du prélèvement par l'écouvillon, celui-ci est placé dans le tube qui contient l'eau nécessaire à la réhydratation du support poreux placé à sa partie apicale. Le tube est ensuite bouché et incubé.

Dans un autre mode de réalisation, le support poreux est intégré dans un pansement, un bandage, une couche culotte ou un emballage alimentaire.

Préférentiellement, le dispositif comprend sous le support poreux une couche inferieure poreuse qui est mise en contact avec l'échantillon à analyser.

Encore préférentiellement, le dispositif comprend également une couche supérieure imperméable transparente sur au moins un partie.

L'invention concerne également l'utilisation d'un dispositif selon l'invention pour détecter et/ou identifier et/ou énumérer au moins un microorganisme cible dans un échantillon susceptible de le contenir.

Avantageusement, l'invention concerne l'utilisation d'un dispositif comprenant une tige à l'extrémité de laquelle ledit support poreux est fixé, en tant qu'écouvillon.

Dans un autre mode de réalisation, l'invention concerne l'utilisation d'un dispositif selon l'invention en tant que pansement.

Suivant un autre mode de réalisation, l'invention concerne également l'utilisation d'un dispositif selon l'invention en tant que couche culotte.

Encore dans un autre mode de réalisation, l'invention concerne l'utilisation d'un dispositif selon l'invention en tant que conditionnement de denrées alimentaires.

On entend par échantillon une petite partie ou petite quantité séparée d'une entité par un acte soustractif dénommé habituellement prélèvement, à des fins d'analyse. L'échantillon peut être d'origine biologique, humaine, animale, végétale ou environnementale. Il peut concerner un produit au cours d'un process industriel ou un produit fini, par exemple alimentaire. Il peut donc correspondre à un prélèvement de fluide biologique (sang total, sérum, plasma, urine, liquide céphalo-rachidien, sécrétion organique), un prélèvement tissulaire ou des cellules isolées. Il peut être d'origine industrielle, soit, selon une liste non exhaustive un prélèvement d'air, un prélèvement d'eau, un prélèvement effectué sur une surface, une pièce ou un produit en cours de traitement ou manufacturé, un produit d'origine alimentaire. Parmi les échantillons d'origine alimentaire, on peut citer de façon non exhaustive un échantillon de produits lactés (yaourts, fromages...), de viande, de poisson, d'oeufs, de fruits, de légumes, d'eau, de boisson (lait, jus de fruits, soda, etc) et les produits constitutifs ou annexes du produit fini. Un échantillon alimentaire peut enfin être issu d'une alimentation destinée aux animaux, telle que notamment des farines animales. Ce prélèvement peut subir préalablement à son analyse une préparation de type enrichissement, extraction, concentration, purification, selon des méthodes connues de l'homme du métier.

Au sens de la présente invention, le terme micro-organisme recouvre les bactéries à Gram positif ou Gram négatif, les levures, les moisissures, les amibes et plus généralement, les organismes unicellulaires, invisibles à l'oeil nu, qui peuvent être manipulés et multipliés en laboratoire.

Selon un mode préféré de réalisation de l'invention, le micro-organisme est une bactérie, gram négative ou positive, ou une levure.

A titre de bactéries Gram positives, on peut citer les bactéries des genres suivants : *Enterococcus, Streptococcus, Lactobacillus, Bifidobacterium, Staphylococcus, Bacillus, Listeria, Clostridium, Mycobacteria, Nocardia, Corynebacteria, Micrococcus et Deinococcus.*

A titre de bactéries Gram négatives on peut citer les bactéries des genres suivants : *Salmonella, Escherichia coli et Pseudomonas.*

A titre de levures, on peut citer les levures des genres suivants : *Candida, Cryptococcus, Saccharomyces et Trichosporon.*

*A titre de moisissures, on peut citer les moisissures des genres suivants : Aspergillus, Penicillium, Cladosporium .*

On entend par support poreux, un volume à porosité adaptée, sous forme de tissus ou de non-tissé présentant un réseau fibreux ou filamenteux, de mousse à porosité ouverte. Il s'agit d'un support tridimensionnel dans lequel les particules du milieu réactionnel ont pénétré dans une zone donnée du support poreux dans toute son épaisseur.

Le support peut être à base de divers composés absorbants à très fort pouvoir de rétention d'eau tels que la viscose, rayonne, le coton, les fibres cellulosiques naturelles ou modifiées chimiquement comme la carboxy-méthyl cellulose, les polymères chimiques absorbants ou super-absorbants tels que sels de polyacrylate, copolymère acrylate/acrylamide. De façon préférentielle, le support poreux aura une masse surfacique comprise entre 50g/m² et 150g/m², préférentiellement entre 90g/m² et 110g/m².

Par milieu réactionnel, on entend un milieu comprenant tous les éléments nécessaires à la survie et/ou à la croissance des microorganismes. Ce milieu réactionnel peut soit servir uniquement de milieu de révélation, soit servir de milieu de culture et de révélation. Dans le premier cas, la culture des microorganismes a lieu préalablement, et, dans le second cas, le milieu réactionnel constitue également le milieu de culture. Ainsi, le milieu réactionnel du dispositif selon l'invention est un milieu de révélation et/ou un milieu de culture.

On entend par milieu de révélation, tout milieu contenant une molécule capable de se coupler avec les micro-organismes ou les partenaires de liaison desdits micro-organismes et permettant, par leurs propriétés de transduction (fluorescence, coloration, radioactivité, etc.) de révéler la présence desdits micro-organismes. Cette révélation de la présence des micro-organismes cibles peut être notamment obtenue par visualisation (à l'oeil nu) ou lecture optique d'une coloration ou d'une fluorescence sur tout ou partie du support.

On entend par milieu de culture, un milieu comprenant tous les éléments nécessaires à la survie et/ou à la croissance de micro-organismes. En pratique, l'homme du métier choisira le milieu de culture en fonction des micro-organismes cibles, selon des critères parfaitement connus et à la portée de cet homme de l'art.

Le milieu réactionnel selon l'invention peut contenir d'éventuels additifs comme par exemple : des peptones, un ou plusieurs facteurs de croissance, des hydrates de carbone, un ou plusieurs agents sélectifs, des tampons, colorants, un ou plusieurs gélifiants, des hydrogels, agent visqueux, etc...

Préférentiellement, le milieu réactionnel du dispositif selon l'invention comprend au moins un gélifiant dont la quantité est comprise entre 1mg/cm² et 2mg/cm². L'agent gélifiant peut être choisi parmi les gélifiants bien connus de l'homme du métier tels que l'agar, l'agarose, les poloxamères, la gomme guar, le xanthane.

Ainsi, le support poreux et les gélifiants permettent de limiter la diffusion des substrats et des micro-organismes et participent à la formation de colonies microbiennes isolées.

On entend par poudre, des particules ayant une granulométrie de 1 à 200 micromètres. Une micronisation des composés minoritaires tels que les substrats, antibiotiques, est possible pour parfaire l'homogénéisation de la poudre.

Par «au moins un micro-organisme cible», l'on entend, au sens de la présente invention, au moins un micro-organisme que l'on souhaite détecter et/ou identifier et/ou dénombrer.

Au sens de la présente invention, la définition de la « sensibilité de détection » est identique à celle communément admise dans l'état de la technique, à savoir la capacité à donner un résultat positif (apparition d'une réaction colorée et/ou fluorescente) lorsque la souche bactérienne cible est présente dans l'échantillon.

### Description des dessins :

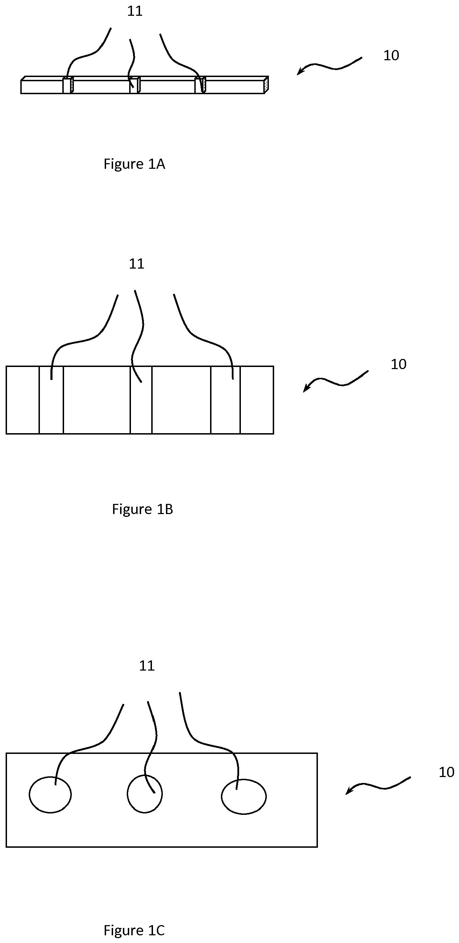

La figure 1 est une représentation schématique de l'invention nullement limitative.
la figure 1 représente de façon schématique un support poreux (10) compris dans un dispositif selon l'invention comprenant une pluralité de milieux réactionnels (11) au niveau de différentes zones, chacune de ces zones ayant alors du milieu réactionnel distribué dans toute l'épaisseur du support.
La figure 1b correspond à une vue du dessus du support poreux (10) selon l'invention.
La figure 1c correspond à une vue de dessus selon un autre mode de réalisation où le support poreux est imprégné par des milieux réactionnels au niveau de zones formant des ronds en surface, chacune de ces zones ayant alors du milieu réactionnel distribué dans toute l'épaisseur du support.

**Exemple 1** : préparation d'un support poreux selon l'invention, comprenant de la poudre de milieu de culture distribuée de façon homogène au sein du support.

### Matériel :

- Supports non tissés 95 g/m² (ref 95NN81, sca Life) de dimension 25 cm² et d'épaisseur 2 mm (avant calandrage) :
- Milieu de culture deshydraté ChromID CPS 3, 0,13g (600-04595, bioMerieux) + xanthane 0.06g (ref 4452073479, Alliance gum pharma)

### Protocole :

0,2 g de poudre est saupoudré sur le support de façon homogène puis le support est placé entre deux électrodes en appliquant un voltage de 3200V/mm pendant 15 secondes à une humidité relative de comprise entre 35 et 45%.

Le support est calandré à 60°C en appliquant une pression de 3.10⁵ Pa/cm².

Les supports sont ainsi traversés par la poudre de milieu de culture.

**Exemple 2 :** préparation d'un support poreux selon l'invention, comprenant de la poudre de milieu réactionnel distribuée de façon homogène au sein du support et de la poudre de milieu réactionnel distribuée uniquement en surface.

### Matériel :

- Supports non tissés 95 g/m²de dimension 25 cm² et d'épaisseur 2 mm (avant calandrage) (ref 95NN81, sca Life).
- Milieu 1 : milieu de culture deshydraté ChromID CPS 3(0,13g)(600-04595, bioMerieux) + xanthane 0.06g (ref 4452073479, Alliance gum)
- Milieu 2 : milieu de culture deshydraté ChromID CPS 3(0,13g)(600-04595, bioMerieux) + xanthane 0.06g (ref 4452073479, Alliance gum)+ imipenème monohydrate micronisé (sp2129, Merck and Co)

Une première face est imprégnée de milieu de réactionnel de manière homogène selon le protocole de l'exemple 1. Puis le support est retourné et la deuxième face est imprégnée avec un milieu réactionnel comprenant un antibiotique l'imipenème à l'aide d'un champ électrique plus faible de 1050 V/mm pendant 15 secondes pour que la poudre pénètre moins et reste plus en surface. Le calandrage est effectué comme décrit à l'exemple 1.

**Exemple 3 :** préparation d'un support poreux selon l'invention, comprenant de la poudre de milieu réactionnel distribuée de façon homogène au sein du support et de la poudre de milieu réactionnel distribuée en gradient au sein du support.

### Matériel identique à l'exemple 2.

Une première face est imprégnée de milieu 1 de manière homogène selon le protocole de l'exemple 1. Puis le support est retourné, et l'on imprègne la deuxième face avec le deuxième milieu avec le même champ électrique fort (3169V/mm).

On obtient donc un gradient d'imprégnation du deuxième milieu réactionnel à l'intérieur d'une répartition homogène du premier milieu réactionnel.

**Exemple 4 :** préparation d'un support poreux selon l'invention, comprenant différentes poudres de milieux réactionnels distribuées au sein du support en deux couches.

Une première face est imprégnée avec le milieu réactionnel de manière homogène avec un champ électrique moyen (1408 V/mm). Puis le support est retourné, et l'on imprègne la seconde face avec un deuxième milieu réactionnel avec le même champ électrique moyen (1408 V/mm).

On obtient donc deux couches distinctes de milieux réactionnels et toute l'épaisseur du support poreux comprend de la poudre de milieu réactionnel.

**Exemple 5 :** Détection de *Escherichia coli* et *Pseudomonas aeruginosa* avec un dispositif selon l'invention comprenant en tant qu'antibiotique l'imipenème à 1 mg/l.

### Matériel :

1. Réactifs utilisés
   - Gélose CPS3 (bioMérieux ref. 43549, 22002)
   - Chrom ID CPS3 milieu sec (bioMerieux, ref. 600-04595)
   - Flacon Tryptone sel 9 ml, (AES ref. 111499, lot 327601)
   - Xanthane (Alliance Gum FF Pharma lot FF 2524429) granulométrie <75µm
   - Imipenème (bioMérieux INC Ref 066259-1)
2. Souches utilisées
   - *E.coli* ATCC 25922, CMI (Concentration minimale inhibitrice) de 0.12mg/ml
   - *Pseudomonas aeruginosa* ATCC 27853, CMI 2 mg/ml

### Méthode :

Pour réaliser les milieux gélosés, un litre d'eau est rajouté à la prise d'essai du milieu sec soit 38,3g pour ChromID CPS3. Le milieux sec est alors dissous sous agitation, porté à ébullition puis stérilisé par autoclavage. Après refroidissement du milieu gélosé à 55°C, l'imipenème stérilisé par filtration est rajouté à la concentration de 1mg/l.

Pour réaliser une imprégnation suivant la présente invention du milieu secs Chrom ID CPS3, une prise d'essai du milieu sec correspondant à la fabrication d'un litre de milieu est réalisée soit 26g, prise d'essai à laquelle est rajoutée le xanthane (20g) et l'imipenème (1g). L'ensemble des constituants est ensuite mélangé dans un turbula®. Les supports poreux sont ensuite imprégnés par les poudres des milieux de cultures réalisées comme décrites ci-dessus et stérilisés par rayon gamma entre 10 et 17 kGy.

### Résultats :

### Gélose ChromID CPS3

| | Concentrations UFC/ml de départ | ChromID CPS3 sans Antibiotique | | ChromID CPS3 avec Imipenème 1mg/l | |
|---|---|---|---|---|---|
| | | | | | |
| | | UFC | UFC/ml | UFC | UFC/ml |
| *E.coli* ATCC 25922 | 10³ | 67/56/88 | 703 | 0/0/0 | 0 |
| | 10⁵ | ≥ 300 colonies | - | 0/0/0 | 0 |
| *Pseudomonas aeruginosa* ATCC 27853 | 10³ | 122/105/105 | 1107 | 69/61/73 | 677 |
| | 10⁵ | ≥ 300 colonies | - | ≥ 300 colonies | - |

UFC : Unité Formant Colonies

### Support poreux selon l'invention

| | Concentrations UFC/ml de départ | ChromID CPS3 sans Antibiotique | | ChromID CPS3 avec Imipenème 1mg/l | |
|---|---|---|---|---|---|
| | | UFC | UFC/ml | UFC | UFC/ml |
| *E.coli* ATCC 25922 | 10³ | 28/32/32 | 307 | 0/0/0/0/0/0 | 0 |
| | 10⁵ | ≥ 300 colonies | - | 0/0/0/0/0/0 | 0 |
| *Pseudomonas aeruginosa* ATCC 27853 | 10³ | 49/57/61 | 556 | 40/53/38/39/46/56 | 453 |
| | 10⁵ | ≥ 300 colonies | - | ≥ 300 colonies | - |

### Conclusion

Deux souches ont été testées, *E.coli* ATCC 25922 (CMI 0.12) et *Pseudomonas aeruginosa* ATCC 27853 (CMI 2mg/l).

Les résultats obtenus avec les supports poreux selon l'invention et avec les milieux gélosés ChromID CPS3 sont concordants avec la croissance de la souche *Pseudomonas aeruginosa* ATCC 27853 ayant une CMI de 2mg/l en présence d'imipenème à 1 mg/L et l'absence de croissance de la souche *E.coli* ATCC 25922 ayant une CMI de 0,12 mg/l en présence d'imipenème à 1 mg/L.

**Exemple 6 :** Détection de *Proteus* et de *Pseudomonas* avec un dispositif selon l'invention comprenant en tant qu'antibiotique le Ciprofloxacine à 1,5 mg/l.

### Matériel :

- Base Mueller Hinton 2 poudre (bioMérieux, Ref 8301143, lot 1002661760)
- Agar Américain (ROKO SA Ref 11000301)
- Agar Européen (SETEXAM Ref TMN2)
- Ciprofloxacine (HCL TOKU-E COMPANY Ref C032)
- Gélose ChromID CPS3 (bioMérieux Ref 43549, 22002)
- Chrom ID CPS3 milieu sec (bioMérieux Ref 600-04595)
- Flacon Tryptone sel 9 ml (AES ref 111499, lot 327601)
- Gélose MH2 ((Muller Hinton 2) pré-coulées (bioMérieux Ref:43301)
- Gélose TSA (bioMérieux Ref : 43011)
- Xanthane (Alliance Gum FF Pharma lot FF 2524429) granulométrie <75µm
- Ciprofloxacine (HCL ref: 00743041)

Les supports poreux ont été imprégnés par les milieux suivants :
- MH2 (Muller Hinton 2) + xanthan alliance gum pharma
- MH2 + xanthan alliance gum pharma + 1.5 mg/L ciprofloxacine
- MH2 + xanthan alliance gum pharma
- CPS3 + xanthan alliance gum pharma
- CPS3 + xanthan alliance gum pharma + 1.5 mg/L ciprofloxacine

Souches testées :
- Proteus mirabilis API 8803099, ADM AP3, CMI : 0,125
- Proteus mirabilis API 8803080, ADM JS10, CMI : 0,25
- Proteus mirabilis API 9406037, collection bioMérieux, CMI : 4
- Proteus vulgaris API 8803017, ADM CQ11, CMI : 0,125
- Pseudomonas aeruginosa API 9405061, collection bioMérieux, CMI : 0,125
- Pseudomonas aeruginosa API 7509005, ATCC 25853, CMI : 0,5
- Pseudomonas aeruginosa API 9410075 CMI: 16
- Pseudomonas aeruginosa API 9405063 CMI: 4

### Méthode :

Pour réaliser les milieux gélosés un litre d'eau est rajouté à la prise d'essai du milieu sec soit 38,3g pour ChromID CPS3 et 41,57 g pour le milieu Muller Hinton2. Le milieu sec est ensuite dissous sous agitation magnétique, porté à ébullition puis stérilisé par autoclavage. Après refroidissement du milieu gélosé à 55°C, la ciprofloxacine stérilisée par filtration est rajoutée au milieu gélosé à la concentration de 1,5mg/l.

Pour réaliser une imprégnation suivant la présente invention des milieux secs MH2 et Chrom ID CPS3, la prise d'essai du milieu sec correspondant à la fabrication d'un litre de milieu est réalisée soit 26g pour ChromID CPS3 et 26,07g pour le milieu Muller Hinton2, prise d'essai à laquelle est rajoutée le xanthane (20g) et si besoin la ciprofloxacine (1,5g). L'ensemble est ensuite mélangé dans un turbula®. Les supports poreux sont ensuite imprégnés des poudres des milieux de cultures comme décrits ci-dessus et stérilisés par rayon gamma entre 10 et 17 kGy.

### Résultats :

| SOUCHES | CMI | Milieu gélosé MH2 | Milieu gélosé MH2 + Ci 1,5mg/l | Imprégnation support poreux avec MH2 | Imprégnation support poreux avec MH2 + Ci 1,5 mg/l |
|---|---|---|---|---|---|
| *Proteus mirabilis* API 8803099 | 0,032 | ++ | -- | ++ | -- |
| *Proteus mirabilis* API 8803080 | 0,38-0,5 | ++ | -- | ++ | -- |
| *Proteus mirabilis* API 9406037 | 8-12 | ++ | ++ | ++ | ++ |
| *Proteus vulgaris* API 8803017 | 0,125 | ++ | -- | ++ | -- |
| *P. aeruginosa* API 9405061 | 0,25 | ++ | -- | ++ | -- |
| *P. aeruginosa* API 7509005 | 0,25 | ++ | -- | ++ | -- |
| *P. aeruginosa* API 9410075 | sup 32 | ++ | ++ | ++ | ++ |
| *P. aeruginosa* API 9405063 | 6-8 | ++ | ++ | ++ | ++ |

| SOUCHES | CMI | Milieu gélosé ChromID CPS3 | Milieu gélosé ChromID CPS3 + Ci 1,5mg/l | Imprégnation support poreux avec ChromID CPS3 | Imprégnation support poreux avec ChromID CPS3 + Ci 1,5 mg/l |
|---|---|---|---|---|---|
| *Proteus mirabilis* API 8803099 | 0,032 | ++ | -- | ++ | -- |
| *Proteus mirabilis* API 8803080 | 0,38-0,5 | ++ | -- | ++ | -- |
| *Proteus mirabilis* API 9406037 | 8-12 | ++ | ++ | ++ | ++ |
| *Proteus vulgaris* API 8803017 | 0,125 | ++ | -- | ++ | -- |
| *P. aeruginosa* API 9405061 | 0,25 | ++ | -- | ++ | -- |

| | | | | | |
|---|---|---|---|---|---|
| *P. aeruginosa* API 7509005 | 0,25 | ++ | -- | ++ | -- |
| *P. aeruginosa* API 9410075 | sup 32 | ++ | ++ | ++ | ++ |
| *P.aeruginosa* API 9405063 | 6-8 | ++ | ++ | ++ | ++ |

### Conclusions

Dans cet exemple les milieux gélosés Muller Hinton et ChromID CPS3 avec ou sans ciprofloxacine à 1,5 g/L ont été comparés aux support poreux suivant la présente invention imprégnés par les mêmes milieux avec ou sans ciprofloxacine à 1,5 g/l pour trois souches de *Proteus mirabilis,* une souche de *Proteus vulgaris* et quatre souches de *Pseudomonas aeruginosa.* Toutes ces souches étaient de CMI encadrant la valeur 1,5 mg/l de ciprofloxacine. Les souches de CMI inférieures à 1,5 mg/l (*Proteus mirabilis* :API 8803099, *Proteus mirabilis* :API 8803080, *Proteus vulgaris* : API 8803017, *Pseudomonas aeruginosa* : API 9405061, *Pseudomonas aeruginosa* : API 7509005) ne présentent pas de croissance sur milieux gélosés MH2 + Substrats + ciprofloxacine (1,5 mg/l) et ChromID CPS3 additionnés en ciprofloxacine (1,5 mg/l). De la même manière ces souches dites sensibles à la ciprofloxacine pour une CMI de 1,5 mg/l ne présentent pas non plus de croissance sur les supports poreux imprégnés avec les milieux ChromID CPS3 et MH2+Substrat en présence de ciprofloxacine à 1,5 mg/l. Il y a donc concordance entre les résultats obtenus sur milieux gélosés et sur supports poreux imprégnés suivant la présente invention. Toutes ces souches présentent une croissance sur milieux gélosés MH2+Substrat, ChromID CPS3, supports poreux imprégnés par milieux MH2+Substrat et ChromID CPS3.

Les souches de CMI supérieures à 1,5 mg/l (*Proteus mirabilis* : API 9406037, *Pseudomonas aeruginosa* : API 9410075, *Pseudomonas aeruginosa*: API 940506) poussent sur tous les milieux gélosés ou imprégnés avec ou sans ciprofloxacine (1,5g/l). Ces résultats confirment qu'il est possible de réaliser des milieux de culture suivant la présente invention contenant de faibles quantités de principes actifs comme des substrats chromogéniques ou des antibiotiques.

## Revendications

1. Procédé de détection et/ou identification et/ou énumération, d'au moins un microorganisme cible dans un échantillon susceptible de le contenir, comprenant les étapes suivantes :
(a) fournir un dispositif pour la détection et/ou l'identification et/ou l'énumération de microorganismes comprenant un support poreux comprenant de la poudre d'un milieu de culture avec au moins un antibiotique et/ou au moins un composé thermosensible tel qu'un substrat enzymatique ou métabolique, dans toute son épaisseur, ledit support poreux ayant été imprégné à sec dans toute son épaisseur par ledit milieu de culture,
(b) mettre en contact un échantillon avec le support poreux, le milieu de culture étant réhydraté par un échantillon aqueux ou un volume approprié de liquide lorsque l'échantillon n'est pas aqueux ou insuffisamment aqueux.
(c) incuber le dispositif,
(d) détecter et/ou identifier et/ou énumérer la ou les colonies de microorganismes au sein du support poreux, lorsque les microorganismes recherchés sont présents dans l'échantillon.

2. Procédé selon la revendication 1 comprenant une étape préalable de préparation, dilution ou concentration de l'échantillon.

3. Procédé selon l'une quelconque des revendications 1 ou 2, dans lequel l'échantillon est mis en contact avec la partie inférieure du support poreux.

4. Procédé selon l'une quelconque des revendications 1 ou 2, dans lequel l'étape b) se fait par prélèvement de l'échantillon à l'aide du support poreux.

5. Dispositif comprenant un support poreux comprenant au moins un milieu de culture sous forme de poudre réparti dans toute l'épaisseur du support poreux, ledit milieu de culture comprenant au moins un antibiotique, et/ou au moins un composé thermosensible tel qu'un substrat enzymatique ou métabolique, ledit support poreux ayant une épaisseur comprise entre 0,5 à 2mm et étant calandré.

6. Dispositif selon la revendication 5 **caractérisé en ce qu'**au moins un milieu de culture sous forme de poudre est réparti dans l'épaisseur du support poreux de façon homogène.

7. Dispositif selon la revendication 5 **caractérisé en ce qu'**au moins un milieu de culture sous forme de poudre est réparti graduellement dans l'épaisseur du support poreux.

8. Dispositif selon la revendication 7 **caractérisé en ce qu'**il comprend au moins deux milieux de culture différents sous forme de poudre répartis en au moins deux couches, ledit support comprenant à un point donné de l'épaisseur l'un ou l'autre milieu de culture.

9. Dispositif selon l'une quelconque des revendications 5 à 8 dans laquelle le milieu de culture comprend au moins un gélifiant dont la quantité est comprise entre 1mg/cm² et 2mg/cm².

10. Dispositif selon l'une quelconque des revendications 5 à 9 dans laquelle la quantité de milieu de culture imprégnée dans le support poreux est comprise entre 0.10mg/cm³ et 0.1g/cm³ de préférence entre 0.01 g/cm³ et 0.09 g/cm³.

11. Dispositif comprenant une pluralité de support poreux tels que décrits dans les revendications 5 à 10.

12. Pansement comprenant le dispositif selon l'une quelconque des revendications 5 à 10.

13. Couche culotte comprenant le dispositif selon l'une quelconque des revendications 5 à 10.

14. Emballage alimentaire comprenant le dispositif selon l'une quelconque des revendications 5 à 10.

15. Ecouvillon comprenant le dispositif selon l'une quelconque des revendications 5 à 10.

16. Utilisation d'un dispositif selon l'une quelconque des revendications 5 à 10 pour détecter et/ou identifier et/ou énumérer au moins un microorganisme cible dans un échantillon susceptible de le contenir.

## Patentansprüche

1. Verfahren zur Detektion und/oder Identifizierung und/oder Zählung mindestens eines Zielmikroorganismus in einer Probe, die ihn enthalten kann, das die folgenden Schritte umfasst:
(a)Liefern einer Vorrichtung für die Detektion und/oder Identifizierung und/oder Zählung von Mikroorganismen, die einen porigen Träger umfasst, der Pulver eines Nährmediums mit mindestens einem Antibiotikum und/oder mindestens einer wärmeempfindlichen Verbindung, wie einem enzymatischen oder metabolischen Substrat, in seiner gesamten Stärke umfasst, wobei der porige Träger trocken in seiner gesamten Stärke durch das Nährmedium imprägniert wurde,
(b)Inberührungbringen einer Probe mit dem porigen Träger, wobei das Nährmedium durch eine wässrige Probe oder ein zweckdienliches Flüssigkeitsvolumen rehydriert wird, wenn die Probe nicht wässrig oder unzureichend wässrig ist,
(c)Inkubieren der Vorrichtung,
(d)Detektieren und/oder Identifizieren und/oder Zählen der Kolonie oder Kolonien von Mikroorganismen innerhalb des porigen Trägers, wenn die gesuchten Mikroorganismen in der Probe vorhanden sind.

2. Verfahren nach Anspruch 1, das einen vorausgehenden Schritt der Vorbereitung, Verdünnung oder Konzentration der Probe umfasst.

3. Verfahren nach einem der Ansprüche 1 oder 2, wobei die Probe mit dem unteren Teil des porigen Trägers in Berührung gebracht wird.

4. Verfahren nach einem der Ansprüche 1 oder 2, wobei der Schritt b) durch Entnahme der Probe mit Hilfe des porigen Trägers erfolgt.

5. Vorrichtung, die einen porigen Träger umfasst, der mindestens ein Nährmedium in Pulverform umfasst, das in der gesamten Stärke des porigen Trägers verteilt ist, wobei das Nährmedium mindestens ein Antibiotikum und/oder eine wärmeempfindliche Verbindung, wie ein enzymatisches oder metabolisches Substrat, umfasst, wobei der porige Träger eine Stärke zwischen 0,5 und 2 mm umfasst und kalandriert ist.

6. Vorrichtung nach Anspruch 5, **dadurch gekennzeichnet, dass** mindestens ein Nährmedium in Pulverform in der Stärke des porigen Trägers homogen verteilt ist.

7. Vorrichtung nach Anspruch 5, **dadurch gekennzeichnet, dass** mindestens ein Nährmedium in Pulverform graduell in der Stärke des porigen Trägers verteilt ist.

8. Vorrichtung nach Anspruch 7, **dadurch gekennzeichnet, dass** sie mindestens zwei unterschiedliche Nährböden in Pulverform umfasst, die in mindestens zwei Schichten verteilt sind, wobei der Träger an einer gegebenen Stelle der Stärke das eine oder das andere Nährmedium umfasst.

9. Vorrichtung nach einem der Ansprüche 5 bis 8, wobei das Nährmedium mindestens ein Geliermittel umfasst, dessen Menge zwischen 1 mg/cm² und 2 mg/cm² liegt.

10. Vorrichtung nach einem der Ansprüche 5 bis 9, wobei die Menge an imprägniertem Nährmedium in dem porigen Träger zwischen 0,10 mg/cm³ und 0,1 g/cm³, bevorzugt zwischen 0,01 g/cm³ und 0,09 g/cm³ liegt.

11. Vorrichtung, die eine Mehrzahl poriger Träger wie in den Ansprüchen 5 bis 10 beschrieben umfasst.

12. Verband, der die Vorrichtung nach einem der Ansprüche 5 bis 10 umfasst.

13. Höschenwindel, die die Vorrichtung nach einem der Ansprüche 5 bis 10 umfasst.

14. Nahrungsmittelverpackung, die die Vorrichtung nach einem der Ansprüche 5 bis 10 umfasst.

15. Flaschenbürste, die die Vorrichtung nach einem der Ansprüche 5 bis 10 umfasst.

16. Gebrauch einer Vorrichtung nach einem der Ansprüche 5 bis 10, um mindestens einen Zielmikroorganismus in einer Probe, die ihn enthalten kann, zu detektieren und/oder zu identifizieren und/oder zu zählen.

## Claims

1. A method for the detection and/or identification and/or enumeration of at least one target microorganism in a sample liable to contain it, comprising the following steps:
(a) providing a device for the detection and/or identification and/or enumeration of microorganisms comprising a porous support comprising culture medium powder with at least one antibiotic and/or at least one heat-sensitive compound such as an enzymatic or metabolic substrate, throughout its thickness, said porous support having been dry-impregnated throughout its thickness by said culture medium,
(b) placing a sample in contact with the porous support, the culture medium being rehydrated by an aqueous sample or a suitable volume of liquid when the sample is not aqueous or is insufficiently aqueous,
(c) incubating the device,
(d) detecting and/or identifying and/or enumerating the colony or colonies of microorganisms within the porous support, when the microorganisms sought are present in the sample.

2. The method as claimed in claim 1, comprising a prior step of preparation, dilution or concentration of the sample.

3. The method as claimed in either one of claims 1 and 2, wherein the sample is placed in contact with the lower portion of the porous support.

4. The method as claimed in either one of claims 1 and 2, wherein step b) is carried out by taking the sample using the porous support.

5. A device comprising a porous support comprising at least one culture medium in powder form distributed throughout the thickness of the porous support, said culture medium comprising at least one antibiotic, and/or at least one heat-sensitive compound such as an enzymatic or metabolic substrate, said porous support having a thickness of between 0.5 and 2 mm and being calendered.

6. The device as claimed in claim 5, **characterized in that** at least one culture medium in powder form is homogeneously distributed through the thickness of the porous support.

7. The device as claimed in claim 5, **characterized in that** at least one culture medium in powder form is distributed in a graduated manner through the thickness of the porous support.

8. The device as claimed in claim 7, **characterized in that** it comprises at least two different culture media in powder form distributed in at least two layers, said support comprising, at a given point through the thickness, one or the other culture medium.

9. The device as claimed in any one of the claims 5 to 8, wherein the culture medium comprises at least one gelling agent, the amount of which is between 1 mg/cm² and 2 mg/cm².

10. The device as claimed in any one of the claims 5 to 9 wherein the amount of culture medium impregnated in the porous support is between 0.10 mg/cm³ and 0.1 g/cm³, preferably between 0.01 g/cm³ and 0.09 g/cm³.

11. The device comprising a plurality of porous supports as described in claims 5 to 10.

12. A bandage comprising the device as claimed in any one of claims 5 to 10.

13. A sanitaire pad comprising the device as claimed in any one of claims 5 to 10.

14. A packaging for foodstuffs comprising the device as claimed in any one of claims 5 to 10.

15. A swab comprising the device as claimed in any one of claims 5 to 10.

16. The use of a device as claimed in any one of claims 5 to 10 for detecting and/or identifying and/or enumerating at least one target microorganism in a sample liable to contain it.
